# EUROPEAN PATENT APPLICATION

(11) **EP 2 774 651 A1**
(43) Date of publication of application: **10.09.2014**
(21) Application number: 12845520.1
(22) Date of filing: 06.03.2012
(51) Int. Cl.: A61N 1/32, A61N 1/36

(54) **ELECTROSTIMULATION DEVICE AND ELECTROSTIMULATION DEVICE SYSTEM**

(30) Priority: 31.10.2011 JP 2011238451
(71) Applicant: OG Giken Co., Ltd., Okayama-City, Okayama 703-8261 (JP)
(72) Inventor: HIRATA Toshifumi, Okayama-City Okayama 703-8261 (JP); KAJIWARA Toshimasa, Okayama-City Okayama 703-8261 (JP)
(74) Representative: Ter Meer Steinmeister & Partner
(86) International application number: PCT/JP2012/055613
(87) International publication number: WO 2013/065331

(57) **Abstract**

The invention provides an electrostimulation device comprising: an electrostimulator 14 configured to apply electrical stimulation in accordance with a therapy mode through an electrode to a paralyzed region in a patient; a therapy condition setter 22 configured to set a therapy condition including the therapy mode; a display 24 configured to display at least the therapy condition set by the therapy condition setter 22; an information communicator 16 configured to transmit information including the therapy condition to at least another electrostimulation device; and a controller 19 configured to control these structural elements.

## Description

### TECHNICAL FIELD

The invention relates to electrostimulation devices and electrostimulation device systems, more particularly to electrostimulation devices and electrostimulation device systems suitably used for therapeutic rehabilitation of the motor functions of paralyzed regions (including muscle weakness regions) in patients with cerebrovascular diseases such as cerebral hemorrhage wand cerebral infarction, orthopedic diseases, Parkinson's disease, and pinocerebellar degeneration.

### BACKGROUND ART

The related art reveals electrostimulation devices characterized in that electrical stimulation is applied to nerves and muscle groups for relief of pains and improvements in muscular atrophy. Some of these devices are configured to detect a myoelectric signal in a paralyzed region, and apply electrical stimulation to the paralyzed region based on the detected myoelectric signal for therapeutic rehabilitation of the motor function of the paralyzed region. The applicant developed and commercialized an electrostimulation device system provided with an electrostimulation device (electrostimulator) and a program device connected to the electrostimulation device with a wire (Non-patent document 1). The electrostimulation device applies electrical stimulation to a patient's paralyzed region through electrodes. Through the wire connection, the program device sets in the electrostimulation device a therapy condition in accordance with a therapy mode to apply electrical stimulation with a strength that conforms to a myoelectric potential detected in the paralyzed region.

According to the electrostimulation device system, the electrostimulation device is available for therapeutic rehabilitation of the motor function of the paralyzed region after the therapy condition is set in the electrostimulation device by the program device. The program device per se is not configured to function as the electrostimulation device. This leaves medical institutions, such as hospitals and clinics, no choice but to purchase the electrostimulation device and the program device both which are, in fact, sold in a pair. Thus, the electrostimulation device system, in terms of its purchasing cost, is an economically inefficient device.

When, for example, a doctor sets a therapy condition under a therapy mode to treat a patient in another location or site, the doctor needs to carry the whole electrostimulation device system (electrostimulation device and program device both). Thus, a further problem of the conventional system is poor portability.

When, for example, a patient takes the electrostimulation device home for therapeutic use by himself or with an aid from his family after a therapy condition is set in the device by his doctor in a hospital, the doctor is responsible for supervising whether the patient is precisely following the doctor' s instructions (such as time and frequency of the therapy).

The electrostimulation device system, however, is not equipped with a device configured to manage therapy history information of the electrostimulation device. To keep track of the therapy performed on the patient at his home, the doctor has to rely on the patient's self-reported information, and it is very difficult to determine whether the therapy is really performed as exactly instructed by the doctor. This raised a strong demand for an electrostimulation device system wherein the therapy history information can be accurately grasped and managed.

### RELATED ART DOCUMENT

### NON-PATENT DOCUMENT

Non-patent Document 1: OG GIKEN Equipment for Physiotherapy and Rehabilitation GENERAL CATALOG, page 35 to 38, 2010-11, OG GIKEN CO., LTD.

### SUMMARY OF THE INVENTION

### PROBLEM TO BE SOLVED BY THE INVENTION

The invention was accomplished to solve the conventional problems. The invention has a first object to provide an inexpensive and readily portable electrostimulation device which is solely capable of performing a therapy through the application of electrical stimulation and setting a therapy condition of another electrostimulationdevice. The invention further has a second object to provide an electrostimulation device system that enables doctors to accurately grasp and manage therapy history information of another electrostimulation device by using the electrostimulation device.

### MEANS FOR SOLVING THE PROBLEM

To achieve these objects, the invention provides an electrostimulation device, including an electrostimulator configured to apply electrical stimulation in accordance with a therapy mode to a paralyzed region in a patient through electrodes, a therapy condition setter configured to set a therapy condition such as the therapy mode, a display configured to display at least the therapy condition set by the therapy condition setter, an information communicator configured to transmit the therapy condition to at least another electrostimulation device, and a controller configured to control these structural elements.

According to the conventional electrostimulation device system, the program device simply designed to set the therapy condition of the electrostimulation device cannot be used for any therapies. According to the invention, one electrostimulation device can solely exert two functions; setting a therapy condition of another electrostimulation device (electrostimulation device disclosed in the Background Art) and performing a therapy by using the electrostimulator provided therein.

Then, medical institutions no longer have to purchase an electrostimulation device system where two devices are combined; electrostimulation device and program device. Unless they need an extra electrostimulation device, they can choose to buy one electrostimulation device. This reduces the purchasing cost, improving the economic aspect of the device. A further advantage is a better portability because it is just the electrostimulation device that needs to be transported whenever the therapy is performed in another location or site.

Preferably, the therapy mode includes at least a first therapy mode where the electrostimulator applies the electrical stimulation to the paralyzed region with an intensity that conforms to a myoelectric potential detected in the paralyzed region, and a second therapy mode where the electrostimulator applies the electrical stimulation to any region but the paralyzed region in the patient or a region in any person but the patient with an intensity that conforms to a myoelectric potential detected in the region.

This allows to choose between the first mode and the second mode; which of them is more suitable for the patient's medical condition and outcomes of the therapy, offering multiple therapeutic options.

Preferably, a power source of electrostimulation device is a chargeable or non-chargeable battery.

The conventional program device needs to be connected to a commercial power source to be driven. This program device, therefore, can only be used in limited places near the commercial power sources. Because the power source according to the invention is a chargeable or non-chargeable battery, the electrostimulation device is usable anywhere unless the battery power runs out.

Preferably, the electrostimulation device further includes a battery voltage detector configured to detect a battery voltage of the battery, where in decreases of the battery voltage are broadcasted based on a detection result obtained by the battery voltage detector.

This helps doctors and patients to readily know whether the battery voltage of the electrostimulation device is decreasing, thereby preventing such an unfavorable event that the battery power runs out during the therapy due to failure to replace or recharge the battery, forcing the therapy to be interrupted.

Preferably, the electrostimulation device further includes a storage configured to store therein at least the therapy condition in a readable manner, wherein the storage can store therein a plurality of the therapy conditions.

In the case where only one electrostimulation device is available for more than one patient, the therapy conditions for different patients can be separately set in the device. This saves the trouble of resetting the therapy condition all over again for each patient, helping the therapy to be efficiently performed.

Advantageously, the invention is further characterized in that the electrostimulation device and another electrostimulation device are provided, wherein the electrostimulation device receives therapy history information from the another electrostimulation device through the information communicator and displays the received therapy history information on the display.

When a doctor extracts the therapy history information from the another electrostimulation device into the electrostimulation device and displays the information on the display, the doctor can readily know his patient's therapy history information.

Preferably, the therapy history information contains at least one of informations obtained in the another electrostimulation device; accumulated therapy time which is a total therapy time when the therapy is performed, number of times when the electrical stimulation is applied to the paralyzed region, and accumulated stimulation-applying time which is a total time when the electrical stimulation is applied to the paralyzed region.

A doctor can obtain the therapy history information of a patient who underwent home therapy as soon as the doctor checks the accumulated therapy time, number of times when the electrical stimulation is applied, and accumulated stimulation-applying time. Then, the doctor can readily grasp details of the home therapy, whether the therapy is really performed as exactly instructed by the doctor.

Advantageously, the invention is further characterized in that the electrostimulation device and another electrostimulation device are provided, wherein the another electrostimulation device has an information communicator configured to transmit and receive information to and from the electrostimulation device, a storage configured to store therein the information received by the information communicator, and a controller configured to control these structural elements.

According to the structure, the information set in the electrostimulation device, such as the therapy condition, can be extracted from the electrostimulation device and stored in the another electrostimulation device. When, for example, it is decided for some reason to use the another electrostimulation device after the therapy condition is already set in the electrostimulation device, this technical feature can save the trouble of setting the same therapy condition in the another electrostimulation device.

Preferably, the another electrostimulation device can receive the therapy condition from the electrostimulation device and store therein the received therapy condition.

The electrostimulation device may be owned by and kept at a medical institution, in which case the therapy condition is extracted from the electrostimulation device and stored in the another electrostimulation device. Then, the another electrostimulation device can be therapeutically used at a patient's home without his visiting the medical institution.

Preferably, a part of or all of the received therapy condition can be altered or cannot be altered on the another electrostimulation device.

A patient, who uses the another electrostimulation device where no alteration is possible, is unable to discretionally alter the therapy condition set in the device. This prevents the patient from receiving the therapy under any inappropriate therapy condition at home in the absence of medical professionals. Then, the therapy can be suitably performed with safety and effectiveness ensured at all times.

On the other hand, the another electrostimulation device configured to alter the therapy condition can be therapeutically used in a medical institution under the therapy condition suitably altered by a therapy provider. Furthermore, when a patient who uses the another electrostimulation device at home makes minor changes to the therapy condition depending on his medical condition, the f inely-turned therapy is suitably performed.

Preferably, the another electrostimulation device has a body formed in a portable size.

As well as an improved portability of the another electrostimulation device, a patient can always carry the body with him so that the therapy can be readily performed anytime in any location or site.

Preferably, power sources of the electrostimulation device and the another electrostimulation device are chargeable or non-chargeable batteries, wherein the electrostimulation device has a DC-DC converter configured to regulate a battery voltage of the battery to a predetermined voltage value, the another electrostimulation device has a DC-DC converter configured to regulate a battery voltage of the battery to a predetermined voltage value, and the DC-DC converters regulate the battery voltages of the batteries to an equal predetermined voltage value.

When, for example, a patient, who underwent the therapy performed by the electrostimulation device in a medical institution, stores the therapy condition of the electrostimulation device in the another electrostimulation device and takes the another device home for therapeutic use, the electrostimulation outputs of these two devices, although the same therapy condition is set therein, may differ by virtue of different types of batteries (for example, the battery of the electrostimulation device is a dry battery, and the battery of the another electrostimulation device is a secondary battery), or the outputs may differ by virtue of decreased battery capacities. The technical feature described in the preceding paragraph prevents these unfavorable events, helping the therapy to be performed even more effectively.

Advantageously, the invention is further characterized in that the electrostimulation device and another electrostimulation device are provided, wherein the another electrostimulation device has an information communicator configured to transmit and receive information to and from the electrostimulation device, the therapy condition is transmitted from the electrostimulation device and received by the another electrostimulation device, and the therapy history information is transmitted from the another electrostimulation device and received by the electrostimulation device by means of the information communicators of the electrostimulation device and the another electrostimulation device, the electrostimulation device is mostly used therapeutically by therapy providers including doctors in medical institutions, the another electrostimulation device is mostly used by patients for home therapy under the guidance of the therapy providers, and the therapy providers can set the therapy condition in the another electrostimulation device and manage the therapy history information by means of the electrostimulation device.

With this technical feature, a doctor can set the therapy condition in the another electrostimulation device by means of the electrostimulation device used in the medical institution. This prevents the patient from receiving the therapy under any inappropriate therapy condition at home in the absence of medical professionals. Then, the therapy can be suitably performed with safety and effectiveness ensured at all times. Moreover, the therapy provider can accurately grasp and supervise by means of the another electrostimulation device whether the patient is therapeutically using the another electrostimulation device at home as exactly instructed. This ensures that the therapy provider can offer an effective therapeutic guidance to the patient.

### OPERATIONAL ADVANTAGES OF THE INVENTION

According to the invention, one electrostimulation device can solely exert two functions; setting the therapy condition of another electrostimulation device and performing the therapy by using the electrostimulator provided therein. Conventionally, medical institutions had no choice but to purchase the electrostimulation device system where the program device and the electrostimulation device are combined, which is no longer necessary. Unless they need an extra electrostimulation device, they can choose to buy one electrostimulation device. This reduces the purchasing cost, improving the economic aspect of the device. A further advantage is a better portability because it is just the electrostimulation device that needs to be transported whenever a doctor performs the therapy in another location or site.

The electrostimulation device system provided by the invention is effectively used in such a situation that a patient or his family uses the another electrostimulation device for home therapy. A doctor, by extracting the therapy history information from the another electrostimulation device into the electrostimulation device, can display the information and readily grasp details of the home therapy, whether the therapy is really per formed as exactly instructed by the doctor.

### BRIEF DESCRIPTION OF THE DRAWINGS

Fig. 1 is a plan view of an electrostimulation device wherein a therapy is performed under a first therapy mode according to a first embodiment of the invention.
Fig. 2 is a plan view of another electrostimulation device according to the first embodiment.
Fig. 3 is a block diagram illustrating an electrical configuration of the electrostimulation device according to the first embodiment.
Fig. 4 is a plan view of the electrostimulation device wherein the therapy is performed under a second therapy mode according to the first embodiment.
Fig. 5 is a perspective view of the another electrostimulation device according to the first embodiment loaded in a charging device.
Fig. 6 is a plan view of a state of communication between the electrostimulation device and the another electrostimulation device according to the first embodiment.
Fig. 7 is an enlarge view of a liquid crystal display of the electrostimulation device when a therapy condition is set therein according to the first embodiment.
Fig. 8 is an enlarge view of the liquid crystal display when the therapy condition set in the electrostimulation is transmitted to the another electrostimulation device according to the first embodiment.
Fig. 9 is an enlarge view of the liquid crystal display of the electrostimulation on which therapy history information of the another electrostimulation device is displayed according to the first embodiment.
Fig. 10 is an illustration of information transmitted and received to and from the electrostimulation device and the another electrostimulation device according to the first embodiment.

### EMBODIMENT FOR CARRYING OUT THE INVENTION

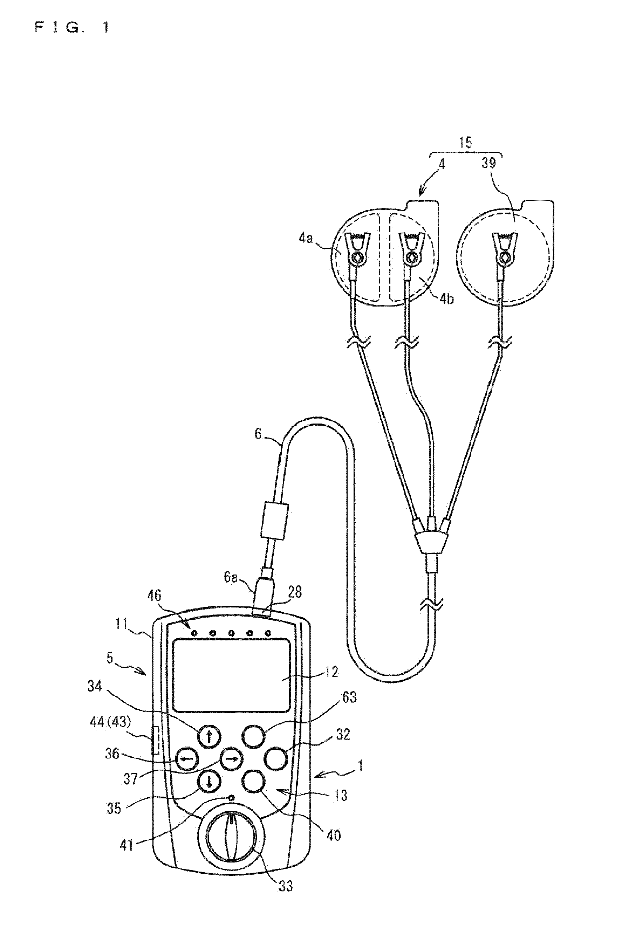

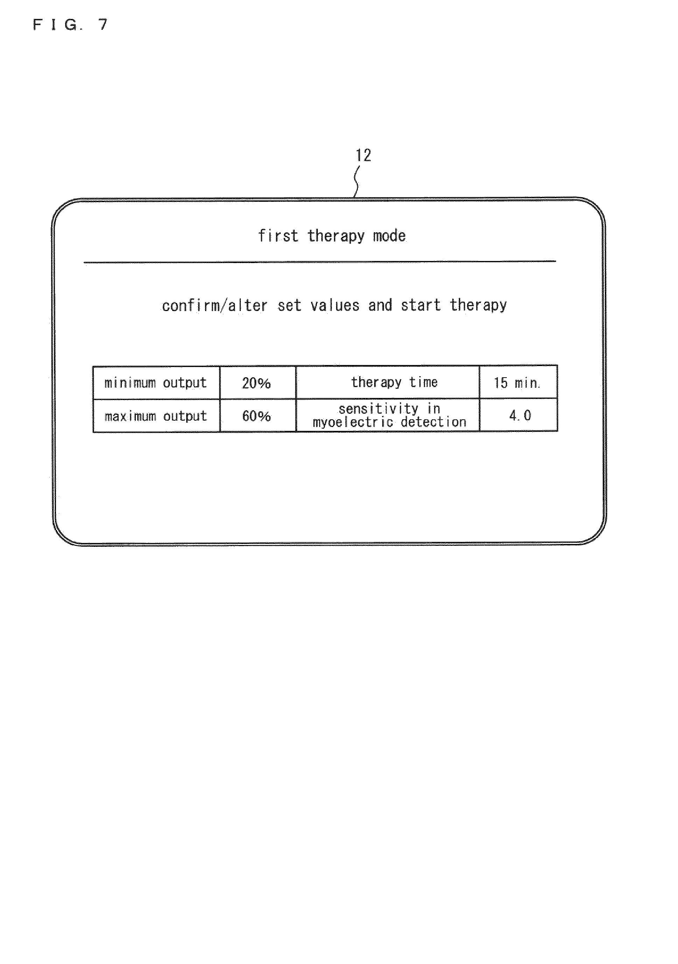

An electrostimulation device 1, another electrostimulation device 2, and an electrostimulation device system 3 according to a first embodiment of the invention are hereinafter described in detail referring to Figs. 1 to 10. The electrostimulation device 1 isa master unit used by therapy providers such as doctors inmedical institutions, for example, hospitals and clinics. The another electrostimulation device 2 is a slave unit mostly used by patients or their families for home therapy under the guidance of therapy providers. The electrostimulation device system 3 is a system where the electrostimulation device 1 and the another electrostimulation device 2 are combined. A plurality of the another electrostimulation device 2 may be combined with one electrostimulation device 1. In a description of this embodiment hereinafter given, the electrostimulation device system 3 has one electrostimulation device 1 and one electrostimulation device 2.

First, structural and technical features of the electrostimulation device 1 are described referring to Figs. 1, 3, 4, and 7 to 9. As illustrated in Figs. 1, 3, and 4, primary structural elements of the electrostimulation device 1 are; a myoelectric detection - electrostimulation electrode 15, an electrostimulation electrode 38, an electrostimulation device body 5, an electrode cable 6, and an electrode cable 29. The myoelectric detection - electrostimulation electrode 15 is stuck onto the skin surface of a paralyzed region in a patient with hemiplegia to detect a myoelectric signal and also to apply electrical stimulation to the paralyzed region. The electrostimulation electrode 38 is stuck onto the skin surface of the paralyzed region to apply electrical stimulation thereto. The electrostimulation device body 5 has a housing 11 that houses therein an electrostimulator 14 configured to feed electrical stimulation to the myoelectric detection - electrostimulation electrode 15 and the electrostimulation electrode 38. The electrode cable 6 and the electrode cable 29 are used respectively to connect the myoelectric detection - electrostimulation electrode 15 and the electrostimulation electrode 38 to the electrical stimulation device body 5.

The myoelectric detection - electrostimulation electrode 15 includes a bipolar electrode 4 with two electrodes 4a and 4b integrally combined, and an electrode 39. These electrodes are hook-type gel electrodes whose back surfaces are stuck onto the patient's skin surface. The hooks of the electrodes 4a, 4b, and 39 are connected to ends of the electrode cable 6 trifurcatedat an intermediate position thereof. A connection plug 6a on the base-end side of the electrode cable 6 is removably inserted in a first output connector 28 formed at a right-hand position on an upper side surface of the housing 11.

The electrodes 4a and 4b are stuck onto the surface of skin above the venters of target muscles. The electrodes 4a and 4b detect a very weak myoelectric potential generated therebetween by the patient's muscular activity. The electrodes 4a and 4b further serve as electrostimulation electrodes to apply electrical stimulation. The electrode 39 is an electrostimulation electrode stuck onto the surface of skin above the venters of muscles targeted for electrical stimulation to apply electrical stimulation to the muscles. The electrode 39 further serves as a ground electrode by which a reference potential is decided to detect the myoelectric potential between the electrodes 4a and 4b.

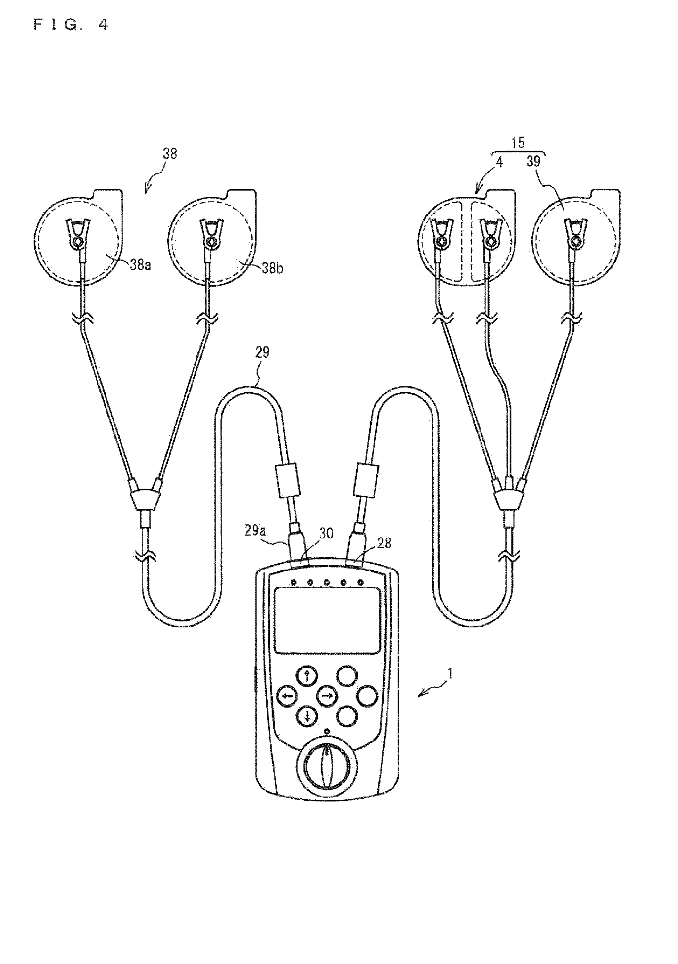

As illustrated in Fig. 4, the electrostimulation electrode 38 with two hook-type gel electrodes 38a and 39a whose back surfaces are stuck onto the patient's skin surface is provided separately from the myoelectric detection - electrostimulation electrode 15. The hooks of the electrodes 38a and 39b are latched on ends of the electrode cable 29 bifurcated at an intermediate position thereof . A connection plug 29a on the base-end side of the electrode cable 29 is removably inserted in a second output connector 30 provided at a left-hand position on the upper side surface of the housing 11.

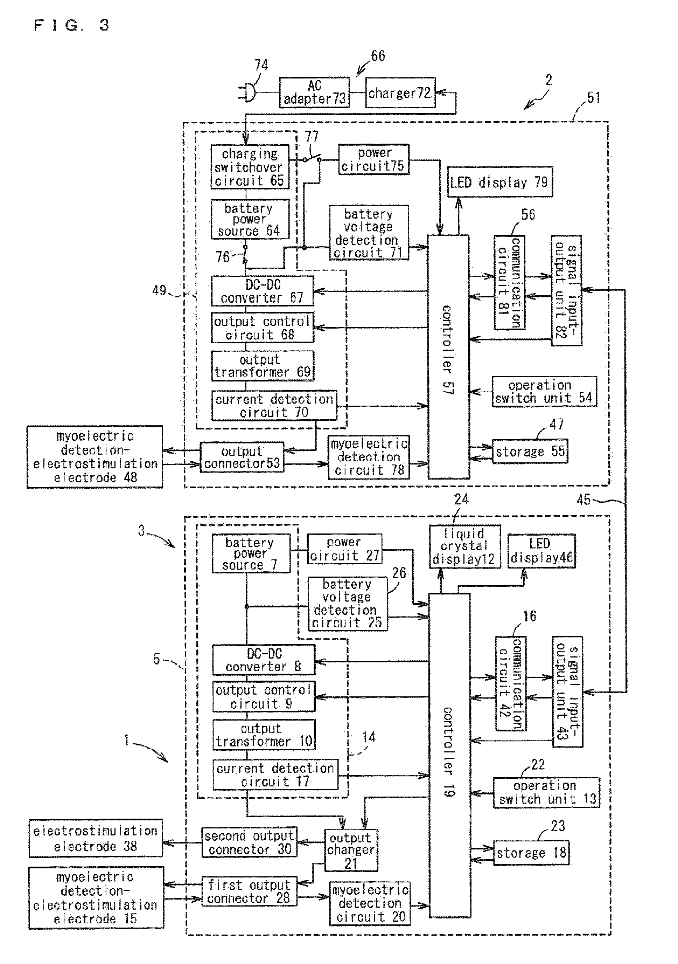

Referring to Figs. 1 and 3, structural elements of the electrostimulation device body 5 are; a housing 11 substantially formed in the shape of rectangular parallelepiped, an operation switch unit 13 (therapy condition setter 22), a storage 18 (storage 23), a liquid crystal display 12 (display 24), an electrostimulator 14 housed in the housing 11, an information communicator 16, and a controller 19. The operation switch unit 13 is provided in a lower part on an upper surface of the housing 11. The operation switch unit 13 includes different switches respectively used to set and alter a therapy condition in accordance with a therapy mode. The storage 18 includes a non-volatile memory (EEPROM) configured to store therein a set therapy condition in a readable manner. The liquid crystal displays 12 (display 24) is provided in an upper part on the upper surface of the housing 11 to display thereon information such as the set therapy condition. The information communicator 16 is configured to transmit and receive the therapy condition and therapy history information to and the another electrostimulation device 2. The controller 19 including a microcomputer is configured to control these structural elements

As illustrated in Fig. 1, the operation switch unit 12 includes the following switches; a power switch 32 to turn on and off a main power source, a regulating knob 33 to regulate an electrostimulation output and a sensitivity in myoelectric detection, an upward switch 34 for upwardmovement of the cursor of the liquid crystal display 12 and to increment set values of the therapy condition, a downward switch 35 for downward movement of the cursor of the liquid crystal display 12 and to decrement set values of the therapy condition, a leftward switch 36 and a rightward switch 37 for leftward and rightward movements of the cursor of the liquid crystal display 12, an enter switch 40tofinalize setting and alteration of the therapy condition, and an operation lock switch 63 to cancel the operations requested by pressing any switches but the power switch 32 and the operation lock switch 63.

The therapy condition of the electrostimulation device 1 includes a therapy mode, therapy time, minimum and maximum electrostimulation outputs, and sensitivity in myoelectric detection, which can be set by manipulating the operation switch unit 13. The therapy mode has two options; first therapy mode, and second therapy mode. One of these modes is selected and set in the device. The maximum and minimum electrostimulation outputs increase and decrease depending on an amount of rotation of the regulating knob 33. Specifically, the cursor is moved to an appropriate column through the manipulation of the switches 34, 35, 36, and/or 37, and the regulating knob 33 is rotated clockwise. Then, the output (%) increases. When the regulating knob 33 is rotated counterclockwise with the cursor placed on the column, the output (%) decreases. Even when the minimum output is set to 0%, the electrical stimulation, though very weak, is still outputted. Similarly, the sensitivity in myoelectric detection increases and decreases depending on an amount of rotation of the regulating knob 33. The sensitivity increases when the regulating knob 33 is rotated clockwise, whereas the sensitivity decreases when the regulating knob 33 is rotated counterclockwise. The therapy time is set by suitably pressing the upward switch 34 or the downward switch 35. The storage 18 is configured to store therein the therapy condition set by manipulating the operating switch unit 13 in a readable manner, where a plurality of therapy conditions can be stored. In the case where one electrostimulation device 1 is only available for more than one patient, different therapy conditions for the plural patients can be separately set in the device.

The electrostimulator 14 has a battery power source 7, a DC-DC converter 8, an output control circuit 9, an output transformer 10, and a current detection circuit 17. The DC-DC converter 8 is configured to boost the voltage of the battery power source 7 and feed the boosted voltage as an output power. The output control circuit 9 is configured to control the voltage inputted from the DC-DC converter 8. The output transformer 10 is configured to boost an output voltage from the output control circuit 9. The current detection circuit 17 is configured to detect an output current from the output transformer 10. An output current signal detected by the current detection circuit 17 is inputted to the controller 19, and the controller 19 accordingly controls the output control circuit 9.

The battery power source 7 has four AA dry batteries (for example, 1.5V) that are serially connected. Though not illustrated in the drawings, the battery power source 7 can be housed in a battery container formed on the back surface of the housing 11. The structure of the battery power source 7 is not necessarily limited to the serially connected four AA dry batteries. It may be arbitrarily decided what type of or how many batteries are used. Other examples are commercial dry batteries such as alkaline, nickel-cadmium, and nickel-hydride batteries, and chargeable batteries such as lithium ion secondary batteries. Further provided is a battery voltage detection circuit 25 (battery voltage detector 26) configured to detect the voltage value of the battery power source 7. When it is detected by the battery voltage detection circuit 25 that the voltage value of the battery power source 7 has declined to a first threshold, the controller 19 broadcasts the decline in voltage to, for example, a therapy provider by displaying the information in the form of an image. When it is detected by the battery voltage detection circuit 25 that the voltage value of the battery power source 7 has declined to a second threshold lower than the first threshold, the controller 19 turns off the power source of the electrostimulation device 1. A reference symbol 27 illustrated in Fig. 3 is a power circuit connected to the battery power source 7 to feed control power to the controller 19.

The myoelectric detection is hereinafter described. At a predetermined frequency (for example, 20 Hz), a bidirectional square wave with a predetermined pulse width (for example, 50 µs) is repeatedly outputted from the output transformer 10 to between the bipolar electrode 4 and the electrode 39, in which three consecutive outputs are counted as a basic output unit. Then, a myoelectric potential at intervals between the repeated outputs (in particular, 8 ms) is detected between the electrode 4a and the electrode 4b. The myoelectric potential detected between the electrodes 4a and 4b is inputted to the myoelectric detection circuit 20. The myoelectric potential is then amplified by an amplifier not illustrated in the drawings to such an intensity that can be recognized by the controller 19 and inputted to the controller 19. The controller 19 arithmetically operates an intensity of the myoelectric potential through signal processes and controls the output control circuit 9 so that the electrical stimulation is outputted with a strength that conforms to the obtained potential intensity. As a result, there occurs an output current flow between the bipolar electrode 4 and the electrode 39, and the electrical stimulation is accordingly applied to the paralyzed region. The electrostimulation output, though applied to the paralyzed region in accordance with the myoelectric potential intensity, is controlled to stay below the maximum output set on the operation switch unit 13. On the other hand, the minimum electrostimulation output set on the operation switch unit 13 is always applied even if the myoelectric potential is not detected by the myoelectric detection circuit 20.

An LED display 46 is provided in the housing 11. The LED display 46 has fiveLEDs laterally spaced at equal intervals in vicinity of an upper edge of the liquid crystal display 12. These LEDs are myoelectric potential level LEDs that are lighted on in response to the myoelectric potential intensity to visually notify the therapy provider or patient of a current level of themyoelectricpotential. The myoelectric potential intensity is divided in six levels from zero to the maximum value. When the myoelectric potential intensity is at the lowest level, none of the five LEDs is lighted on. When the myoelectric potential intensity increases to the second lowest level, a leftmost one of the LEDs is lighted on (level 1). When the myoelectric potential intensity increases to the third lowest level, two leftmost ones of the LEDs are lighted on (level 2). Then, three to five LEDs are lighted on when the myoelectric potential intensity increases to the higher levels (levels 3 to 5) . A reference symbol 41 in Fig. 1 is an output indication LED that is lighted on during the output of the electrical stimulation.

The therapy mode of the electrostimulation device 1 includes a first therapy mode and a second therapy mode. Under the first therapy mode, an output current (electrical stimulation) with an intensity that conforms to the myoelectric potential detected in the patient's paralyzed region is outputted from the electrostimulator 14 to the paralyzed region. Under the second therapy mode, an output current with an intensity that conforms to a myoelectric potential detected in any region but the paralyzed region or a region in anyone but the patient is outputted from the electrostimulator 14 to the paralyzed region. When the therapy is performed under the first therapy mode, the myoelectric detection - electrostimulation electrode 15 alone is used. When the therapy is performed under the second therapy mode, the myoelectric detection - electrostimulation electrode 15 and the electrostimulation electrode 38 are both used.

A flow direction of the output current from the output transformer 10 is automatically changed by an output changer 21 including latching relay 31. The flow direction is changed to and from the first-output-connector-28 side or the second output-connector-30 side. As to the output changer 21, its point of contact is usually connected so that the output current is outputted from the first-output-connector-28 side. When the second therapy mode is selected as the therapy mode and set in the device by manipulating the operation switch unit 13, the point of contact is automatically changed so that the output current flows to the second-output-connector-30 side.

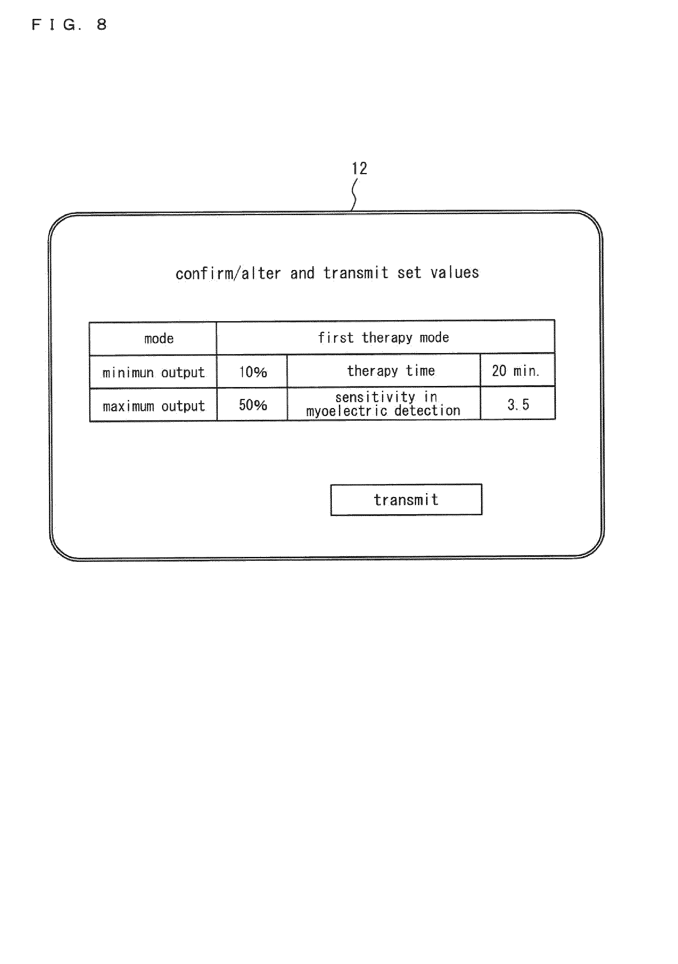

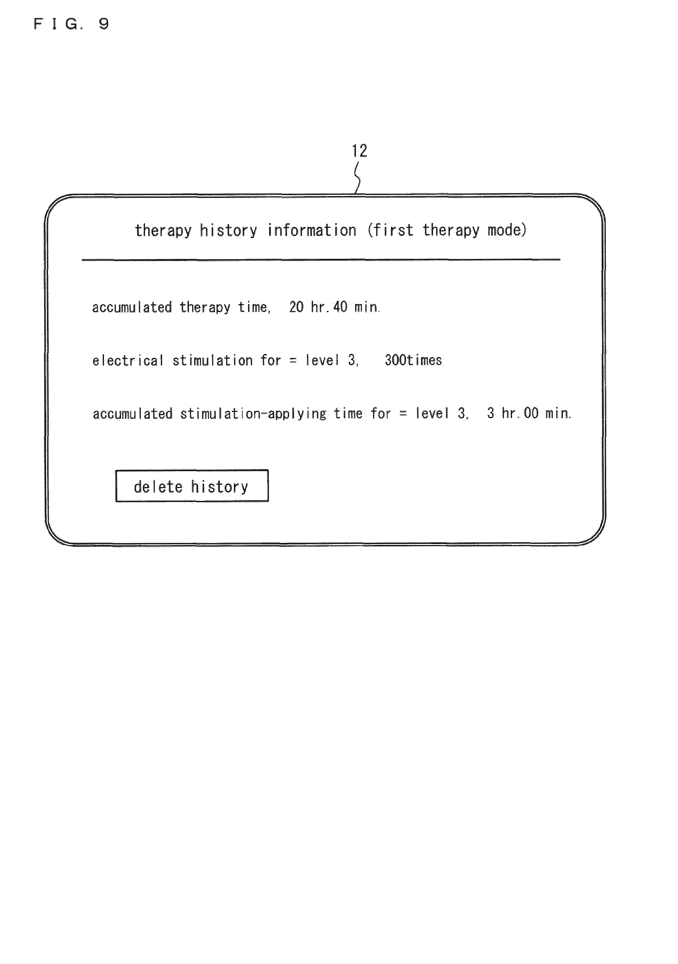

The electrostimulation device 1 is provided with a communication circuit 42 which is an example of the information communicator 16 configured to transmit and receive information to and from the another electrostimulation device 2. The communication circuit 42 includes, for example, a RS-232C converter. The communication circuit 42 is connected to a signal input-output unit 43 (communication connector 44) provided in a center part on a left-side surface of the body 5. The electrostimulation device body 5 is connected to another electrostimulation device body 51 described later with a communication cable 45 connected to the signal input-output unit 43. Through the wire connection, the therapy condition stored in the storage 18 of the electrostimulation device body 5 can be transmitted to the another electrostimulation device body 51 (see Fig. 8), or the therapy history information of the another electrostimulation device 2 can be received and displayed on the liquid crystal display 12 (see Fig. 9).

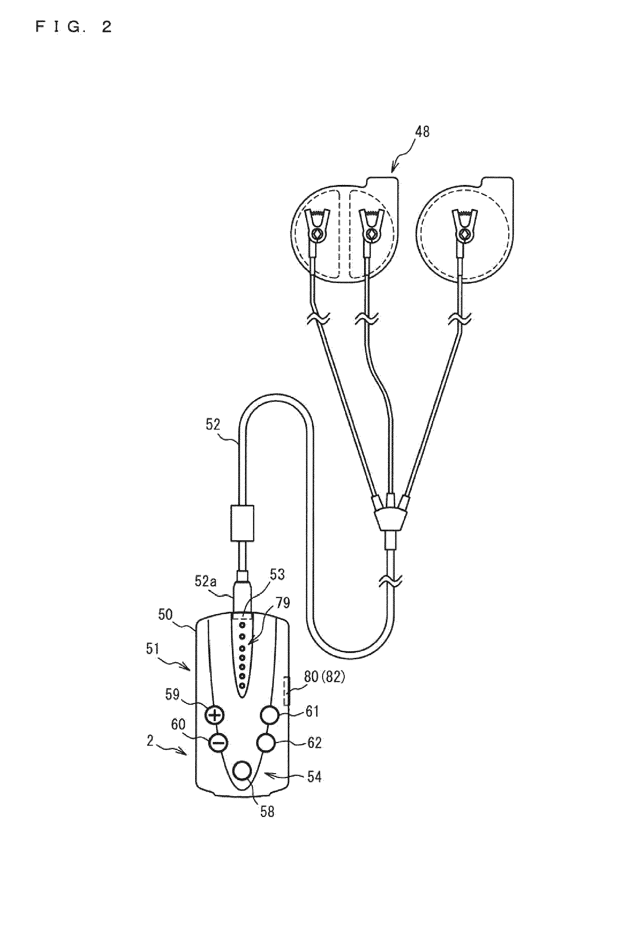

Next, structural and technical features of the another electrostimulation device 2 are described referring to Figs. 2, 3, and 5. Description of the structural elements similar to those of the electrostimulation device 1 is omitted unless particularly necessary. As illustrated in Fig. 2, primary structural elements of the another electrostimulation device 2 are a myoelectric detection - electrostimulation electrode 48, another electrostimulation device body 51, an electrode cable 52, andacharging device 66. The myoelectric detection - electrostimulation electrode 48 is stuck onto the skin surface of a paralyzed region in a patient with hemiplegia to detect a myoelectric signal and apply electrical stimulation to the paralyzed. The another electrostimulation device body 51 includes a housing 50 that houses therein an electrostimulator 49 that feeds electrical stimulation to the myoelectric detection - electrostimulation electrode 48. The electrode cable 52 is used to connect the myoelectric detection - electrostimulation electrode 48 to the another electrical stimulation device body 51. The charging device 66 charges the another electrostimulation device 2.

The myoelectric detection - electrostimulation electrode 48 is configured similarly to the myoelectric detection - electrostimulation electrode 15 of the electrostimulation device 1. A connection plug 52a on the base-end side of the electrode cable 52 is removably inserted in an output connector 53 formed in a center part on an upper side surface of the housing 50.

The another electrostimulation device body 51 has a housing 51, an operation switch unit 54, a storage 55 (storage 47), an electrostimulator 49 provided in the housing 50, an information communicator 56, and a controller 57. The housing 51 is substantially formed in the shape of rectangular parallelepiped. The operation switch unit 54 is provided at a lower part on an upper surface of the housing 50. The storage 55 (storage 47) includes a non-volatile memory (EEPROM) configured to store therein information such as the therapy condition in a readable manner. The information communicator 56 is configured to transmit and receive the therapy condition and therapy history information to and from the electrostimulation device 1. The controller 57 including a microcomputer is configured to control these structural elements.

The housing 50 of the another electrostimulation device 2 is smaller in size than the housing 11 of the electrostimulation device 1 so that the patient can easily carry and use the device for home therapy. Preferably, the housing 50 has a size as small as a 20-cigarette pack. Then, the patient can put the housing 50 in a pocket of his jacket or has the housing 50 stuck to his waist or arm by means of a tightening tool separately provided.

As illustrated in Fig. 2, the operation switch unit 54 includes a sensitivity fine-control switch 59, a sensitivity fine-control switch 60, an output start switch 61, and an output stop switch 62. The sensitivity fine-control switch 59 is used to slightly increase the sensitivity in myoelectric detection. The sensitivity fine-control switch 60 is used to slightly decrease the sensitivity in myoelectric detection. The output start switch 61 is used to start the electrostimulation output. The output stop switch 62 is used to temporarily stop the electrostimulation output. The another electrostimulation device 2 is mostly a home-use therapeutic device used by the patient or his family. To avoid the patient from receiving the therapy under any inappropriate therapy condition at home in the absence of medical professionals, it is neither possible to independently set the therapy condition in the another electrostimulation device body 51 nor alter the therapy condition received from the electrostimulation device body 5 and set in the another electrostimulation device body 51 except for minor changes of the sensitivity in myoelectric detection. The myoelectric potential that can be detected differs depending on the patient's skin condition. The sensitivity in myoelectric detection, therefore, can only be changed (finely controlled) on the another electrostimulation device body 51 by pressing the sensitivity fine-control switch 59 and the sensitivity fine-control switch 60 based on the sensitivity in myoelectric detection received from the electrostimulation device 1. However, it is possible to set the sensitivity in myoelectric detection to be unchangeable on the another electrostimulation device body 5.

At an upper part on the upper surface of the housing 50, there are seven LEDs longitudinally arranged. These LED constitute an LED display 79. Similarly to the LEDs of the electrostimulation device 1, five of these LEDs from the bottom are myoelectric potential level LEDs that are lighted on in response to the detected myoelectric potential intensity. The sixth LED from the bottom is an output indication LED that is lighted on during the output of the electrical stimulation. The LED at the top is a power indication LED that is lighted on when the power source of the another electrostimulation device body 51 is turned on.

The storage 55, which is an example of the storage 47, stores therein the therapy condition transmitted from the electrostimulation device 1 and the therapy history information of the another electrostimulation device 2. The data of the therapy history information to be stored is, for example, accumulated therapy time which is a total therapy time of the another electrostimulation device 2, number of times when the electrical stimulation is applied to the patient's paralyzed region (number of electrical stimulations), and accumulated stimulation-applying time which is a total time when the electrical stimulation is applied to the paralyzed region. The accumulated therapy time is calculated by accumulating time counted by a timer not illustrated in the drawings from the start to finish of the electrostimulation output. The number of electrical stimulations is calculated by counting number of times when the electrical stimulation is applied in response to the myoelectric potential intensities when three or more myoelectric potential level LEDs are lighted on. The accumulated stimulation-applying time is calculated by accumulating time counted by a timer not illustrated in the drawings when the electrical stimulation is applied in response to the myoelectric potential intensities when three or more myoelectric potential level LEDs are lighted on.

A battery power source 64 of the another electrostimulation device 2 is a nickel-hydrogen secondary battery (for example, 2.4 V). Though not illustrated in the drawings, the battery power source 64 is housed in a secondary battery container provided on the back surface of the housing 50. The battery power source 64 is not necessarily limited to the nickel-hydrogen secondary battery. Other batteries, such as a lithium ion secondary battery, may be used. Further provided are a battery voltage detection circuit 71 configured to detect a battery voltage of the battery power source 64, and a power circuit 75 configured to feed control power to the controller 57. When it is detected by the battery voltage detection circuit 71 that the voltage value of the battery power source 64 has declined to a first threshold, the power indication LED is flicked by the controller 57 at predetermined time intervals to broadcast the decline in voltage to the patient. When it is detected by the battery voltage detection circuit 71 that the voltage value of the battery power source 64 has declined to a second threshold lower than the first threshold, the controller 57 turns off the power source of the another electrostimulation device 2.

The structural elements of the electrostimulator 49 of the another electrostimulation device 2 apart from the battery power source 64; a DC-DC converter 67, an output control circuit 68, an output transformer 69, a current detection circuit 70, and a myoelectric detection circuit 78, are configured similarly to those of the electrostimulation device 1. The battery power source 64 and the DC-DC converter 67 are electrically connected to each other by a point-of-contact B switch 76. When, for example, the therapy condition set in the electrostimulation device 1 for a patient is directly transmitted to and stored and used in the another electrostimulation device 2, there may be variability between the electrostimulation outputs from the two devices respectively provided with different battery power sources. To avoid such variability, the DC-DC converter 67 boosts the voltage value of the battery voltage source 64 to a predetermined voltage value (for example, 7.0 v) equal to the voltage value boosted by the DC-DC converter 8 of the electrostimulation device 1.

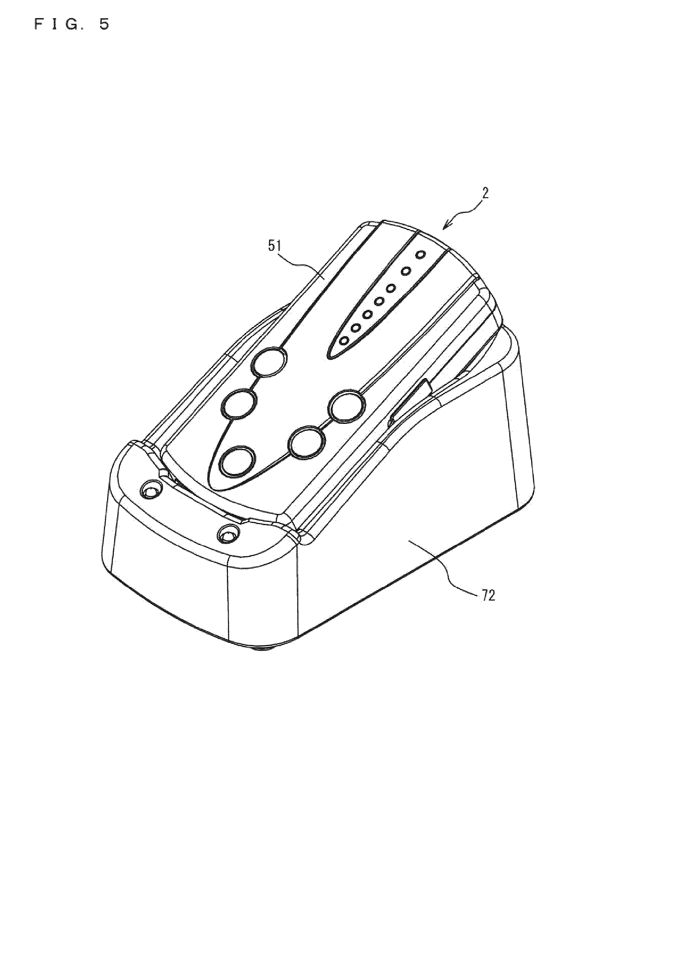

A charging switchover circuit 65 is housed in the housing 50, and a point-of-contact A switch 77 is provided between the charging switchover circuit 65 and the power circuit 75. The charging device 66 includes a charger 72 and an AC adapter 73. When a power code plug 74 of AC adapter 73 connected to the charger 72 is inserted in the socket of a commercial power source, the another electrostimulation device body 51 starts to be charged. As illustrated in Fig. 5, the charger 72 is a cradle-type battery charger where the another electrostimulation device body 51 is chargeable when loaded therein.

When the another electrostimulation device body 51 loaded in the charging device 66 starts to be charged, the point-of-contact B switch 76 is open, and the output control circuit 68 and the battery power source 64 are disconnected from each other. Then, the point-of-contact A switch 77 is closed, and the commercial power source feeds power to the controller 57 through the power circuit 75. When the another electrostimulation device body 51 is being charged, the patient may accidentally touch the switches. To avoid that, the operations of any switches but a power switch 58 are not accepted.

The another electrostimulation device 2 has a communication circuit 81 which is an example of the information communicator 56 configured to transmit and receive information to and from the electrostimulation device 1. The communication circuit 81 includes a RS-232C converter. The communication circuit 81 is connected to a signal input-output unit 82 (communication connector 80) provided in a center part on a right-hand surface of the body 51. The another electrostimulation device body 51 is connected to the electrostimulation device body 5 with a communication cable 45 connected to the signal input-output unit 82. Then, the therapy condition stored in the storage 18 of the electrostimulation device body 5 can be received and stored in the storage 55, or the therapy history information stored in the storage 55 can be transmitted to the electrostimulation device body 5.

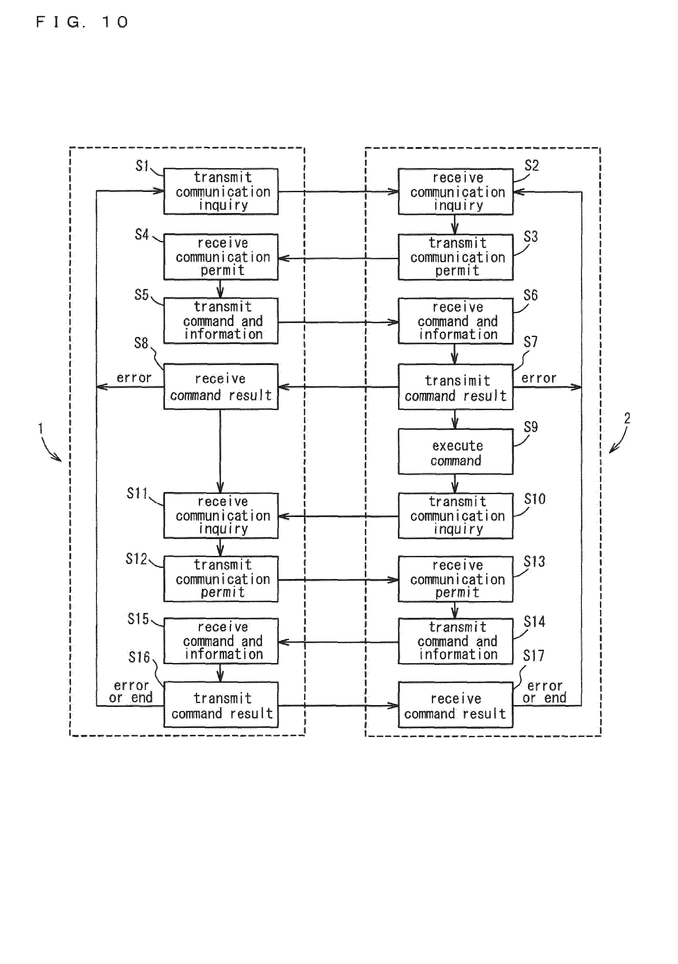

The therapy condition and the therapy history information are transmitted and received to and from the electrostimulation device 1 and the another electrostimulation device 2 according to a communication format illustrated in Fig. 10. First, a description is given to a process to transmit the therapy condition set in the electrostimulation device 1 to the another electrostimulation device 2. A communication inquiry-related signal is transmitted from the electrostimulation device 1 to the another electrostimulation device 2 (S1). The another electrostimulation device 2 receives the (communication inquiry-related signal (S2), and then transmits a communication permit-related signal to the electrostimulation device 1 (S3). The electrostimulation device 1 receives the communication permit-related signal (S4), and then transmits information of set values related to the therapy condition stored in the storage 18 as information to be transmitted along with a command to transmit the information of set values related to the therapy condition to the another electrostimulation device 2 (S5). The another electrostimulation device 2 receives the command and information of set values (S6), and then transmits a command result-related signal to the electrostimulation device 1 (S7). Also, the another electrostimulation device 2 executes the command, in particular, writes the received information of set values in the storage 55 (S9). The electrostimulation device 1 receives the command result-related signal transmitted from the another electrostimulation device 2 (S8). In such an event that the information of set values is mistakenly rewritten owing to an error caused by noise during the transmission of the command and information of set values, the another electrostimulation device 2, in S7, transmits an error-related command result to the electrostimulation device 1. The another electrostimulation device 2 then returns to the communication inquiry receiving step (S2). The electrostimulation device 1 that received the error-related command result in S8 returns to the communication inquiry transmitting step (S1) to restart the communication inquiry (S1).

S10 to S17 are steps of checking whether the information of set values transmitted from the electrostimulation device 1 is correctly written in the storage 55 of the another electrostimulation device 2. After the execution of the command in S9, the another electrostimulation device 2 transmits a communication inquiry-related signal to the electrostimulation device 1 (S10), and the electrostimulation device 1 receives the communication inquiry-related signal (S11). The electrostimulation device 1 transmits a communication permit-related signal (S12), and the another electrostimulation device 2 receives the communication permit-related signal (S13). The another electrostimulation device 2 reads the command received in S6 and temporarily stored in the controller 57 and also reads the information of set values stored in the storage 55 in S9, and then transmits the read command and information to the electrostimulation device 1 (S14). The electrostimulation device 1 receives the command and information of set values (S15). The controller 19 determines whether these command and information are consistent with the command transmitted in S5 and temporarily stored in the controller 19 and the information of set values transmitted in S5 . When the controller 19 confirms consistency between the compared commands and informations, the electrostimulation device 1 transmits a consistency-confirmed command result to the another electrostimulation device 2 (S16). Then, the electrostimulation device 1 returns to the communication inquiry transmitting step (S1). The another electrostimulation device 2 that received the consistency-confirmed command result (S17) returns to the communication inquiry receiving step (S2). This is completion of the process to transmit the information of set value related to the therapy condition from the electrostimulation device 1 to the another electrostimulation device 2. When the controller 19 detects an error, confirming inconsistency between the compared commands and informations, the electrostimulation device 1 transmits an error-related command result to the another electrostimulation device 2 (S16). Then, the electrostimulation device 1 returns to the communication inquiry transmitting step (S1) to restart the communication inquiry from S1. The another electrostimulation device 2 that received the error-related command result (S17) returns to the communication inquiry receiving step (S2).

Next, a description is given to a process to transmit the therapy history information set in the another electrostimulation device 2 to the electrostimulation device 1. Description of S1 to S4 is omitted since these steps are similar to the steps of transmitting the therapy condition set in the electrostimulation device 1 to the another electrostimulation device 2. The electrostimulation device 1 transmits only a command to request the therapy history information of the another electrostimulation device 2 to the another electrostimulation device 2 (S5). The another electrostimulation device 2 receives the command (S6) and transmits a command result to the electrostimulation device 1(S7). Also, the another electrostimulation device 2 executes the command, an particular, reads the therapy history information stored in the storage 55 and accordingly generates information to be transmitted (S9). When the processes of S10 to S13 are over, the another electrostimulation device 2 transmits the generated therapy history information, as well as the command of the therapy history information received in S6 and temporarily stored in the controller 57, to the electrostimulation device 1 (S14). The electrostimulation device 1 receives the command and therapy history information (S15). After the controller 19 determines whether the received command is consistent with the command transmitted in S5 and temporarily stored in the controller 19, the electrostimulation device 1 transmits a command result (S16). When it is determined by the controller 19 that the two commands are consistent, the electrostimulation device 1 returns to the communication inquiry transmitting step (S1), and displays the therapy history information, an example of which is illustrated in Fig. 9, on the liquid crystal display 12. In the event of a command-inconsistent error, the electrostimulation device 1 returns to the communication inquiry transmitting step (S1) to restart the communication inquiry process from S1. The another electrostimulation device 2 receives an error-related command result (S17) and returns to the communication inquiry receiving step (S2).

To delete the therapy history information, the cursor is placed on a history-deletion column illustrated left below in Fig. 9, and the enter switch 40 is pressed. The therapy history information is deleted as a result of steps described below. After the processes of S1 to S4 are over, in S5, a command to delete the therapy history information is transmitted to the another electrostimulation device 2. The another electrostimulation device 2 receives the command (S6), and transmits a command result to the electrostimulation device 1 (S7). Also, the another electrostimulation device 2 executes the command, in particular, the therapy history information stored in the storage 55 is reset to zero. After the processes of S10 to S13 are over, in S4, the another electrostimulation device 2 transmits a command to delete the therapy history information received in S6 and temporarily stored in the controller 57 to the electrostimulation device 1. The electrostimulation device 1 receives the command (S15), and the controller 19 determines whether the received command is consistent with the command transmitted in S5 and temporarily stored in the controller 19. Then, the electrostimulation device 1 transmits a command result (S16). When it is determined by the controller 19 that the two commands are consistent, the electrostimulation device 1 returns to the communication inquiry transmitting step (S1), and displays the therapy history information with all of data reset to zero on the liquid crystal display 12. In the event of a command-inconsistent error, the electrostimulation device 1 returns to the communication inquiry transmitting step (S1) to restart the communication inquiry process from S1. The another electrostimulation device 2 receives an error-related command result (S17) and returns to the communication inquiry receiving step (S2),

The operation of the electrostimulation device 1 is hereinafter described. In an example described below, a therapy provider performs the therapy under the first therapy mode in a medical institution to a patient with impairment in dorsiflexing joints of hand and extending a hand and fingers. The connection plug 6a of the electrode cable 6 is inserted in the first output connector 28 of the electrostimulation device body 5 to connect the electrode cable 6 to the electrostimulation device body 5. The ends of the electrode cable 6 are latched on the hooks of the electrodes 4a, 4b, and 39 two connect the myoelectric detection - electrostimulation electrode 15 to the electrostimulation device body 5. The therapy provider sticks the electrodes 4a and 4b (bipolar electrode 4) onto the patient's paralyzed region, in particular, centers of dorsiflexor muscle groups in the joints of hand of his forearm and/or of extensor muscle groups of the hand and fingers. The therapy provider further sticks the electrode 39 to one end side (wrist side) of the venters of dorsiflexor muscle groups in the joints of hand of his forearm and/or of extensor muscle groups of the hand and fingers.

The therapy provider then presses the power switch 32 of the electrostimulation device body 5. The therapy provider, by pressing the different switches of the operation switch unit 13 while watching the liquid crystal display 12, sets the therapy mode to the first therapy mode and also sets the therapy condition including the therapy time, maximum and minimum electrostimulation outputs, and sensitivity in myoelectric detection. The sensitivity in myoelectric detection regulated by manipulating the regulating knob 33 is such that all of the five myoelectric potential level LEDs 5 of the LED display 46 are lighted on when the forearm is tightened but are lighted off when the forearm is relaxed. If all of the myoelectric potential level LEDs are not lighted on when the forearm is tightened or the electrostimulation output is felt too weak during the therapy, the sensitivity in myoelectric detection is regulated to higher values. On the other hand, if all of the myoelectric potential level LED are not lighted off when the forearm is relaxed or the electrostimulation output is felt too strong during the therapy, the sensitivity in myoelectric detection is regulated to lower values . The sensitivity in myoelectric detection is regulated so that all of the myoelectric potential level LEDs are lighted off when the forearm is relaxed because noise, if entering the myoelectric detection circuit 20, possibly leads to false recognition that the myoelectric potential, though actually not outputted, is detected, resulting in unexpected electrostimulation output. The therapy condition under the first therapy mode for this patient, if already stored in the storage 18, is read out and set. Under the first therapy mode, its frequency is fixed to 20 Hz, however, may be changeable before the therapy is performed.

Under the first therapy mode, the strength of the electrical stimulation to be applied is decided by the intensity of the myoelectric potential detected between the electrodes 4a and 4b, and it is accordingly unnecessary to regulate the output by using the regulating knob 33. When the forearm is tightened, the myoelectric potential detected between the electrodes 4a and 4b is inputted to the myoelectric detection circuit 20. The controller 19 arithmetically operates an intensity of the myoelectric potential. Then, an output current that conforms to the obtained potential intensity flows between the bipolar electrode 4 and the electrode 39. As a result, the electrical stimulation is applied to the dorsiflexor muscle groups in the joints of hand and/or the extensor muscle groups in the hand and fingers. The electrical stimulation thus applied prompts muscular contraction in the dorsiflexor muscle groups in the joints of hand and/or the extensor muscle groups of the hand and fingers. As a result, the joints of hand is dorsiflexed, and the hand and fingers are extended, which are forcibly encouraged by the electric stimulation. This successfully provides effective rehabilitation of the motor functions of the joints of hand and the hand and fingers. When the preset therapy time passed, the electrostimulation output automatically stops. Then, the therapy ends.

When the voltage value of the battery power source 7 decreased after the therapy is repeatedly performed by the electrostimulation device 1, which is displayed on the liquid crystal display 12. Then, the therapy provider removes the used four AA dry batteries from the battery container of the electrostimulation device body 5 to replace them with new ones.

In an example described below, a therapy provider performs the therapy under the second therapy mode in a medical institution to a patient with impairment in his right or left hand in dorsif lexing its joints of hand and extending the hand and fingers. The second therapy mode is effective for patients with severe paralysis, for whom the myoelectric potential is very difficult to detect in their paralyzed regions. Under this therapy mode, for example, electrical stimulation with a strength that conforms to the myoelectric potential detected in an unaffected region can be applied to a paralyzed region. With this attempt to stimulate the paralyzed region and also the unaffected region, the primary motor area and the supplementary motor area are both activated. This provides a good prospect for contraction of voluntary muscles in the paralyzed region.

The therapy provider inserts the connection plug 29a of the electrode cable 29 in the second output connector 30 of the electrostimulation device body 5 to connect the electrode cable 29 to the electrostimulation device body 5. The ends of the electrode cable 29 are latched on the hooks of the electrodes 38a and 38b to connect the electrostimulation electrode 38 to the electrostimulation device body 5. As a result, the electrostimulation electrode 38, in addition to the myoelectric detection - electrostimulation electrode 15, is connected to the electrostimulation device body 5 (see Fig. 4). The therapy provider sticks the myoelectric detection - electrostimulation electrode 15 to the patient's paralyzed region, in particular, onto venters of dorsiflexor muscle groups in the joints of hand and/or of extensor muscle groups in the hand and fingers associated with the paralyzed region (homonymous muscles). The therapy provider further sticks the electrostimulation electrode 38 onto end sides of the venters of dorsiflexor muscle groups in the joints of hand and/or of extensor muscles in the hand and fingers in the paralyzed region.

The therapy provider presses the power switch 32 of the electrostimulation device body 5. Then, the therapy provider, by pressing the different switches of the operation switch unit 13 while watching the liquid crystal display 12, sets the therapy mode to the second therapy mode and also sets the therapy condition including the therapy time, maximum and minimum electrostimulation outputs, and sensitivity in myoelectric detection. Under the second therapy mode, its frequency is similarly fixed to 20 Hz.

The myoelectric potential of the forearm (unaffected) detected between the electrodes 4a and 4b is inputted to the myoelectric detection circuit 20. The controller 19 arithmetically operates an intensity of the myoelectric potential. Then, an output current that conforms to the obtained potential intensity flows between the electrodes 38a and 38b. As a result, the electrical stimulation is applied to the dorsiflexor muscle groups in the joints of hand or the extensormuscles in the hand and fingers of the paralyzed region.

The second therapy mode is also effective in such a case that the myoelectric potential is detected in the muscles of the paralyzed region targeted for electrical stimulation and non-homonymous muscles, and the therapy is performed in a manner that these detected myoelectric potentials are used as a trigger. For example, the myoelectric potential is detected in muscle groups of shoulder joint, and the electrical stimulation is applied to the dorsiflexor muscle groups in the joint of hand and/or the extensor muscle groups of the hand and fingers with a strength that conforms to the detected myoelectric potential. Another possible option is to detect the myoelectric potential in a region someone other than patient, for example, the therapy provider or the patient' s family member and accordingly apply the electrical stimulation to the patient.

The operation of the another electrostimulation device 2 is hereinafter described. The another electrostimulation device 2 is mostly used by a patient who receives the therapy under a therapy provider's guidance. As described earlier, the therapy condition cannot be independently set in the another electrostimulation device 2. Before the another electrostimulation device body 51 is used, a therapy provider needs to, in a medical institution, connect the another electrostimulation device body 51 to the electrostimulation device body 5 with the communication cable 45 and extracts the therapy condition from the electrostimulation device body 5 into the another electrostimulation device body 51. First, the power source of the electrostimulation device body 5 is turned on. Then, the electrostimulation device body 5 is connected to the another electrostimulation device body 51 with the communication cable 45, and the power source of the another electrostimulation device body 51 is turned on. The therapy provider presses the switches of the electrostimulation device body 5 and reads the therapy condition stored in the storage 18. Fig. 8 illustrates the liquid crystal display 12 of the electrostimulation device 1 immediately before the therapy condition is transmitted to the another electrostimulation device 2. On the display, therefore, information of set values related to the read therapy condition is checked to make any corrections to the values, if necessary. Then, the cursor of the liquid crystal display 12 is placed on a transmission column and the enter switch 40 is pressed to transmit the corrected information. Then, the updated information is transmitted to the another electrostimulation device body 51 through the communication cable 45, signal input-output unit 82, and communication circuit 81. The transmitted information is then stored in the storage 55.

After the myoelectric detection, - electrostimulation electrode 48 is connected to the output connector 53, the myoelectric detection - electrostimulation electrode 48 is stuck onto a predetermined position on the patient's forearm (paralyzed region) as described earlier. The patient, while checking his myoelectric potential on the myoelectric potential level LEDs, presses the sensitivity fine-control switches 59 and 60, if necessary, for minor changes of the sensitivity in myoelectric detection. The patient is unable to discretionally alter the maximum and minimum outputs and the therapy time of the therapy condition on the another electrostimulation device 2, which prevents him from receiving the therapy under any unsuitably altered condition. Therefore, effectiveness of the therapy is not undermined.

After all of preparations for the therapy are done, the output start switch 61 is pressed to start the therapy. The another electrostimulation device body 51 small enough to be carried by the patient can be, for example, put in a pocket of his jacket at all times. The patient is accordingly able to undergo the therapy at home during his usual activities . This is a great advantage for the therapy because the myoelectric potential may be constantly detectable in the patient's paralyzed region unconsciously tightened during the usual activities, and the electrical stimulation is applied to the paralyzed region with a strength that conforms to the detected myoelectric potential. Thus, the therapy (rehabilitation of motor functions) is involuntarily performed to the paralyzed region. When the device is thus carried at all times, the myoelectric detection - electrostimulation electrode 48 is preferably tightly stuck to the patient's body and limbs by means of a tightening tool not to fall off from the skin surface. The DC-DC converter 67 of the another electrostimulation device 2 boosts the voltage value of the battery voltage source 64 to a predetermined voltage value equal to the voltage value boosted by the DC-DC converter 8 of the electrostimulation device to apply the electrical stimulation. This prevents variability between the electrostimulation outputs from the two devices respectively provided with different battery power sources during the therapy performed based on the therapy condition received from the electrostimulation device 1.

The therapy can be interrupted during the therapy time by pressing the output stop switch 62. If the voltage of the battery power source 64 decreases and the power indication LED starts to flicker after the therapy is repeatedly performed by the another electrostimulation device 2, the another electrostimulation device body 51 is loaded in the charger 72 to be recharged as illustrated in Fig. 5. To alter the therapy condition already set in the another electrostimulation device body 51, the another electrostimulation device body 51 is connected to the electrostimulation device body 5 with the communication cable 45 to extract the therapy condition of the electrostimulation device body 5 and store the extracted therapy condition in the another electrostimulation device body 51 (overwritten with the newly stored therapy condition).

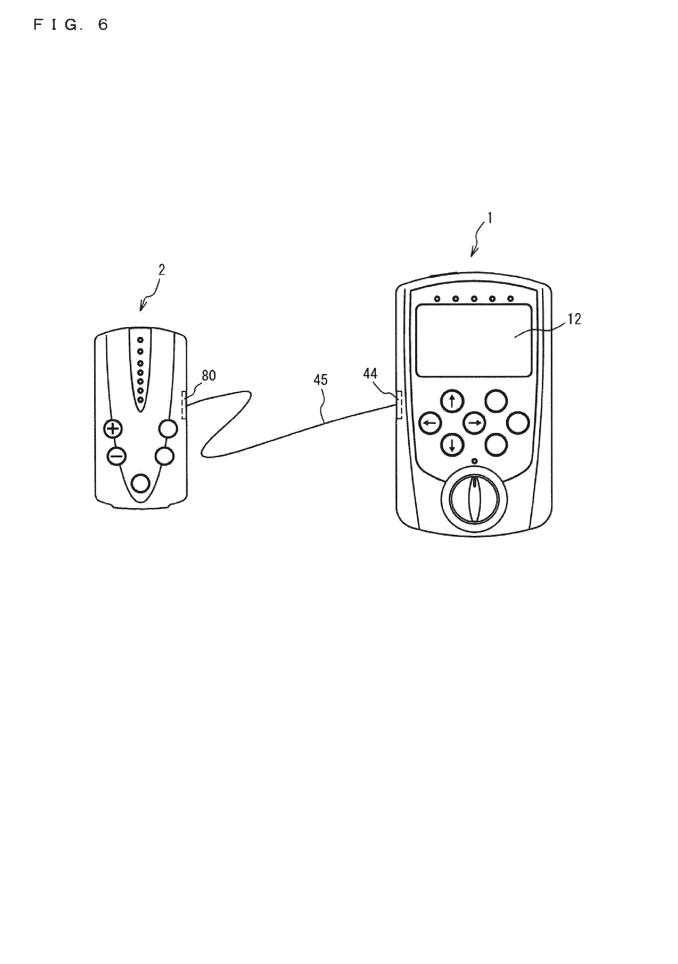

With the ongoing therapy performed by the another electrostimulation device 2, the therapy history information including the accumulated therapy time, number of electrical stimulations, and the accumulated stimulation-applying time is accumulated in the storage 55 of the another electrostimulation device body 51. When the therapy provider wants to know and supervise the current therapy progress, whether the patient is undergoing the therapy at home as precisely instructed, the therapy provider asks his patient to bring the another electrostimulation device 2 when he visits the medical institution. Then, the another electrostimulation device 2 is connected to the electrostimulation device 1 in the medical institution with the communication cable 45 as illustrated in Figs. 1 and 6 to extract the therapy history information from the another electrostimulation device 2 and store the extracted information in the electrostimulation device 1. Then, the stored information is displayed on the liquid crystal display 12. To newly store the therapy history information in the another electrostimulation device 2, the downward switch 35 of the electrostimulation device body 5 is pressed, and the cursor is placed on the history-deletion column displayed on the liquid crystal display 12. Then, the enter switch 40 is pressed so that the therapy history information stored in the storage 55 is reset to zero.

The invention is not necessarily limited to the embodiment described thus far, and may be suitably modified within the scope of the technical concept of the invention. Though not described, in detail or illustrated in the drawings, there are other structural and technical options described below.

To broadcast the voltage decreases of the battery power source 7 in the electrostimulation device 1 may be, in place of indicating such an event on the liquid crystal display 12, an audio message or an alarm sound may be emitted. The indication on the liquid crystal display 12 and the emission of an audio message or an alarm sound may be combined. The remaining battery level of the battery power source 7 may be displayed on the liquid crystal display 12.

Instead of the wired communication using the communication cable 45, the information may be transmitted and received through, for example, infrared radio communication. To transmit and receive the information between the electrostimulation device 1 and the another electrostimulation device 2, any manner of communication format may be adopted as far as the therapy condition and the therapy history information can be communicated therebetween.

The therapy mode to be set in the electrostimulation device 1 is not necessarily limited to two therapy modes; first and second therapy modes. These modes may be combined with, for example, a normal mode that applies electrostimulation outputs independent from the myoelectric potential intensity under a certain condition, in which case one of these three modes is suitably selected and set.

In the embodiment described thus far, the another electrostimulation device 2 receives the therapy condition under the first therapy mode alone from the electrostimulation device 1. The another electrostimulation device body 51 may be configured such that the other therapy modes prepared in the electrostimulation device 1 can be received for the therapy, where appropriate. Moreover, the therapy condition to be set in the another electrostimulation device 2 is not necessarily stored in the storage 18 of the electrostimulation device 1. Other options are acceptable as far as the another electrostimulation device 2 can store therein the therapy condition received from the electrostimulation device 1. After the other therapy modes prepared in the electrostimulation device 1 are received by the another electrostimulation device 2 and available for the therapy, the therapy history information under the other therapy modes is preferably displayed on the liquid crystal display of the electrostimulation device 1.

The another electrostimulation device 2 may be provided with a liquid crystal display similarly to the electrostimulation device 1, so that a therapy provider and/or a patient can check, on the liquid crystal display, the therapy condition received from the electrostimulation device 1 and the therapy history information of the another electrostimulation device 2. In addition to the liquid crystal display, the another electrostimulation device 2 may be provided with an alteration switch used to alter the therapy condition received from the electrostimulation device 1 and/or a password setter that disables a patient to alter the therapy condition, wherein only a therapy provider is allowed to alter a part of or all of the therapy condition displayed on the liquid crystal display. When, for example, the electrostimulation device 1 is occupied and the another electrostimulation device 2 alone is available but can only be used after the therapy condition is altered, a therapy provider does not have to ask his patient to wait until the electrostimulation device 1 becomes available but suitably alters the therapy condition to immediately start the therapy on the patient. When the patients takes the another electrostimulation device 2 for home therapy, the therapy provider sets a password using the password setter to disallow the patient to alter the therapy condition. This ensures that the patient is unable to discretionally alter the therapy condition, improving the usability of the device.

### INDUSTRIAL APPLICABILITY

The invention provides an advantageous technology with high industrial applicability in the field pertinent to electrostimulation devices and electrostimulation device systems suitably used for rehabilitation of the motor functions of paralyzed regions in patients with cerebrovascular diseases such as cerebral hemorrhage and cerebral infarction, orthopedic diseases, Parkinson's disease, and spinocerebellar regeneration.

### DESCRIPTION OF REFERENCE SYMBOLS

1 electrostimulation device
2 another electrostimulation device
3 electrostimulation device system
7 battery power source
8 DC-DC converter
12 liquid crystal display
14 electrostimulator
15 myoelectric detection - electrostimulation electrode
16 information communicator
19 controller
21 output changer
22 therapy condition setter
23 storage
24 display
26 battery voltage detector
31 latching relay
38 electrostimulation electrode
42 communication circuit
44 communication connector
46 communication cable
47 storage
56 information communicator
57 controller
64 battery power source
67 DC-DC converter
80 communication connector
81 communication circuit

## Claims

1. An electrostimulation device, comprising:
an electrostimulator configured to apply electrical stimulation in accordance with a therapy mode to a paralyzed region in a patient through an electrode;
a therapy condition setter configured to set a therapy condition including the therapy mode;
a display configured to display at least the therapy condition set by the therapy condition setter;
an information communicator configured to transmit the therapy condition to at least another electrostimulation device; and
a controller configured to control these structural elements.

2. The electrostimulation device as claimed in claim 1, wherein
the therapy mode includes at least:
a first therapy mode where the electrostimulator applies the electrical stimulation to the paralyzed region with an intensity that conforms to a myoelectric potential detected in the paralyzed region; and
a second therapy mode where the electrostimulator applies electrical stimulation to any region but the paralyzed region in the patient or a region in any person but the patient with an intensity that conforms to a myoelectric potential detected in the region.

3. The electrostimulation device as claimed in claim 1, wherein
a power source of electrostimulation device is a chargeable or non-chargeable battery.

4. The electrostimulation device as claimed in claim 3, further including a battery voltage detector configured to detect a battery voltage of the battery, wherein
decreases of the battery voltage are broadcasted based on a detection result obtained by the battery voltage detector.

5. The electrostimulation device as claimed in one of claims 1 to 4, further including storage configured to store therein at least the therapy condition in a readable manner, wherein
the storage can store therein a plurality of the therapy conditions.

6. An electrostimulation device system, comprising the electrostimulation device as claimed in one of claims 1 to 5, and another electrostimulation device, wherein
the electrostimulation device receives therapy history information from the another electrostimulation device through the information communicator and displays the received therapy history information on the display.

7. The electrostimulation device system as claimed in claim 6, wherein
the therapy history information contains at least one of informations obtained in the another electrostimulation device; accumulated therapy time which is a total therapy time when the therapy is performed, number of times when the electrical stimulation is applied to the paralyzed region, and accumulated stimulation-applying time which is a total time when the electrical stimulation is applied to the paralyzed region.

8. An electrostimulation device system, comprising the electrostimulation device as claimed in one of claims 1 to 5, and another electrostimulation device, wherein the another electrostimulation device includes:
an information communicator configured to transmit and receive information to and from the electrostimulation devices:
a storage configured to store therein the information received by the information communicator; and
a controller configured to control these structural elements.

9. The electrostimulation device system as claimed in claim 8, wherein
the another electrostimulation device can receive the therapy condition from the electrostimulation device and store therein the received therapy condition.

10. The electrostimulation device system as claimed in claim 9, wherein
a part of or all of the received therapy condition can be altered or cannot be altered on the another electrostimulation device.

11. The electrostimulation device system as claimed in one of claims 6 to 10, wherein
the another electrostimulation device has a body formed in a portable size.

12. The electrostimulation device system as claimed in claim 9, wherein
power sources of the electrostimulation device and the another electrostimulation device are chargeable or non-chargeable batteries,
the electrostimulation device has a DC-DC converter configured to regulate a battery voltage of the battery to a predetermined voltage value,
the another electrostimulation device has a DC-DC converter configured to regulate a battery voltage of the battery to a predetermined voltage value, and
the DC-DC converters regulate the battery voltages of the batteries to an equal predetermined voltage value.

13. An electrostimulation device system, comprising the electrostimulation device as claimed in one of claims 1 to 5, and another electrostimulation device, wherein
the another electrostimulation device includes an information communicator configured to transmit and receive information to and from the electrostimulation device,
the therapy condition is transmitted from the electrostimulation device and received by the another electrostimulation device, and the therapy history information is transmitted from the another electrostimulation device and received by the electrostimulation device by means of the information communicators of the electrostimulation device and the another electrostimulation device,
the electrostimulation device is mostly used therapeutically by therapy providers including doctors in medical institutions,
the another electrostimulation device is mostly used by patients for home therapy under the guidance of the therapy providers, and
the therapy providers can set the therapy condition in the another electrostimulation device and manage the therapy history information by means of the electrostimulation device.
